# EUROPEAN PATENT APPLICATION

(11) **EP 4 640 831 A1**
(43) Date of publication of application: **29.10.2025**
(21) Application number: 23906101.3
(22) Date of filing: 22.12.2023
(51) Int. Cl.: C12N 15/113, C12N 15/11

(54) **NUCLEIC ACID MOLECULE AND USE THEREOF**

(30) Priority: 22.12.2022 CN 202211658729
(71) Applicant: Nanjing Curegene Technology Co., Ltd., Nanjing, Jiangsu 211100 (CN)
(72) Inventor: ZHENG, Shasha, Nanjing, Jiangsu 211100 (CN); PAN, Huaye, Nanjing, Jiangsu 211100 (CN); SHAO, Cuiying, Nanjing, Jiangsu 211100 (CN)
(74) Representative: Ström & Gulliksson AB
(86) International application number: PCT/CN2023/141059
(87) International publication number: WO 2024/131946

(57) **Abstract**

Provided are a nucleic acid molecule and a use thereof. The nucleic acid molecule comprises a miRNA backbone nucleic acid molecule for generating a target small molecule RNA. Also provided is an expression vector comprising the nucleic acid molecule. When the miRNA backbone nucleic acid molecule and the expression vector are used for expression of a target small molecule RNA, the expression quantity of the target small molecule RNA in a host cell and the expression quantity of the target small molecule RNA in exosomes secreted by the host cell can be increased.

## Description

### Cross Reference to Related Application

The present application claims the right of priority for Chinese patent application no. CN 202211658729.9 filed on December 22, 2022, which is incorporated herein by reference in its entirety.

### Technical Field

The present invention relates to the field of nucleic acid expression, in particular to the field of small RNA expression.

### Background Art

In recent years, the field of RNA has been well researched, especially in gene and cell therapy, where RNA has shown advantages of high efficiency, good safety, and low cost. For example, RNA interference (RNAi), a phenomenon where a double-stranded RNA induces the specific degradation of a homologous mRNA, has been widely applied in the field of disease treatment. However, due to the instability of RNA within cells and its unsatisfactory expression quantity in cells, the application of RNA, especially small RNA, is greatly limited.

Studies have shown that small RNA can be expressed in cells through an miR backbone structure, which can induce the degradation of passenger strands, thereby significantly reducing off-target effects caused by the passenger strands. Kwan-Ho Chung and his team demonstrated that expressing siRNA by inserting it into the mmu-miR-155 backbone, as compared with the shRNA backbone, results in fewer passenger strands (Polycistronic RNA polymerase II expression vectors for RNA interference based on BIC/miR-155, Nucleic Acids Res. 2006; 34(7): e53). On this basis, it was further optimized by the team of Daniel K. Fowler (Improved knockdown from artificial microRNAs in an enhanced miR-155 backbone: a designer's guide to potent multi-target RNAi, Nucleic Acids Res. 2016 Mar 18; 44(5): e48). It was also found that the mmu-miR-155 backbone can express siRNA *in vivo,* such as in the liver.

In addition, studies have found that siRNA expressed using an miR backbone can enter exosomes and exert effects in other organs via the exosomes. siRNA encapsulated in exosomes can avoid rapid degradation, and with the secretion of exosomes, siRNA can enter the bloodstream and be delivered to more organs and tissues. Studies have shown that the siRNA encapsulated in exosomes in the blood can enter target organs such as lungs, kidneys and colon, and exhibit therapeutic effects in animal models of non-small cell lung cancer.

Nevertheless, the currently used miR backbones still suffer from problems such as low expression quantity and poor therapeutic effects. Therefore, it is necessary to develop a backbone and method capable of increasing RNA expression quantity in cells and exosomes.

### Summary of the Invention

In one aspect, provided herein is an isolated nucleic acid molecule, comprising in a 5'-3' direction:
1) a 5' flanking sequence comprising a sequence as set forth in SEQ ID NO: 1 or a sequence having at least 80% sequence identity thereto;
2) a Loop sequence comprising a sequence as set forth in SEQ ID NO: 3 or a sequence having at least 80% sequence identity thereto; and
3) a 3' flanking sequence comprising a sequence as set forth in SEQ ID NO: 2 or a sequence having at least 80% sequence identity thereto,
wherein the nucleic acid molecule further comprises a coding sequence for a small RNA molecule, and the coding sequence for the small RNA molecule is located between the 5' flanking sequence and the Loop sequence or between the Loop sequence and the 3' flanking sequence.

In some embodiments, the 5' flanking sequence does not comprise the sequence AATTCG at the 3' end, and/or the 3' flanking sequence does not comprise the sequence CACCGGT at the 5' end.

In some embodiments, the nucleic acid molecule further comprises a compensatory sequence, wherein when the coding sequence for the small RNA molecule is located between the 5' flanking sequence and the Loop sequence, the compensatory sequence is located between the Loop sequence and the 3' flanking sequence; when the coding sequence for a small RNA molecule is located between the Loop sequence and the 3' flanking sequence, the compensatory sequence is located between the 5' flanking sequence and the Loop sequence, and the compensatory sequence is at least partially reversely complementary to the coding sequence for the small RNA molecule.

In some embodiments, the compensatory sequence is complementary to at least 80% of bases in the coding sequence for the small RNA molecule; preferably, compared with a sequence fully reversely complementary to the coding sequence for the small RNA molecule, the compensatory sequence lacks any 2-3 consecutive bases at positions 7-12 from the 5' end; more preferably, compared with a sequence fully reversely complementary to the coding sequence for the small RNA molecule, the compensatory sequence lacks bases at positions 9 and 10 from the 5' end.

In some embodiments, in a transcript RNA produced by transcription of the nucleic acid molecule, transcription products of the coding sequence for the small RNA molecule, the Loop sequence and the compensatory sequence are capable of forming a stem-loop structure, wherein the transcription product of the coding sequence for the small RNA molecule and the transcription product of the compensatory sequence form a stem of the stem-loop structure, and the transcription product of the Loop sequence forms a loop of the stem-loop structure.

In some embodiments, the transcript RNA is a pre-miRNA.

In some embodiments, the small RNA molecule is an miRNA or siRNA molecule, preferably 20-24 nt in length.

In some embodiments, the small RNA molecule targets a disease-associated gene expressed in a diseased tissue.

In some embodiments, the disease-associated gene is selected from ataxin-2, SLN, SOD1, ATXN3, C9ORF72, HTT, VEGF, VEGFR, ANG1, ANG2, SARS-CoV-2, HBV, HCV, HIV, HPV, HSV, COX-2, TGF-beta, GBAI, PCSK9, LDL, KRAS, NKRAS, HRAS, EGFR, ATL, MET, PIK3CA, SHP1, SOX2, PTEN, TP53, Yap1, HIF1a, CDK1, CDK4, CDK6, NRF2, beta-Catenin, STAT3, Aurora A, CDH17, CXCR4, IDO1, CSF-1, NF-kB, NOTCH, SOX9, PD-L1, PD1, CTLA4, LAG3, TIGIT, COX-2, TGF-beta, VEGF, VEFGR, BRAF, KRAS, and EGFR genes.

In some embodiments, the small RNA molecule has a sequence as set forth in any one of SEQ ID NOs: 6, 8, 10, 12, 14, 16, 18, 20, 22, 24, 26, 28, 30, 32, 34, 36, 38, 40, 42, 44, 46, 48 or 50.

In some embodiments, 1) the small RNA molecule has a sequence as set forth in SEQ ID NO: 6, and an RNA sequence produced by transcription of the compensatory sequence is as set forth in SEQ ID NO: 7; 2) the small RNA molecule has a sequence as set forth in SEQ ID NO: 8, and an RNA sequence produced by transcription of the compensatory sequence is as set forth in SEQ ID NO: 9; 3) the small RNA molecule has a sequence as set forth in SEQ ID NO: 10, and an RNA sequence produced by transcription of the compensatory sequence is as set forth in SEQ ID NO: 11; 4) the small RNA molecule has a sequence as set forth in SEQ ID NO: 12, and an RNA sequence produced by transcription of the compensatory sequence is as set forth in SEQ ID NO: 13; 5) the small RNA molecule has a sequence as set forth in SEQ ID NO: 14, and an RNA sequence produced by transcription of the compensatory sequence is as set forth in SEQ ID NO: 15; 6) the small RNA molecule has a sequence as set forth in SEQ ID NO: 16, and an RNA sequence produced by transcription of the compensatory sequence is as set forth in SEQ ID NO: 17; 7) the small RNA molecule has a sequence as set forth in SEQ ID NO: 18, and an RNA sequence produced by transcription of the compensatory sequence is as set forth in SEQ ID NO: 19; 8) the small RNA molecule has a sequence as set forth in SEQ ID NO: 20, and an RNA sequence produced by transcription of the compensatory sequence is as set forth in SEQ ID NO: 21; 9) the small RNA molecule has a sequence as set forth in SEQ ID NO: 22, and an RNA sequence produced by transcription of the compensatory sequence is as set forth in SEQ ID NO: 23; 10) the small RNA molecule has a sequence as set forth in SEQ ID NO: 24, and an RNA sequence produced by transcription of the compensatory sequence is as set forth in SEQ ID NO: 25; 11) the small RNA molecule has a sequence as set forth in SEQ ID NO: 26, and an RNA sequence produced by transcription of the compensatory sequence is as set forth in SEQ ID NO: 27; 12) the small RNA molecule has a sequence as set forth in SEQ ID NO: 28, and an RNA sequence produced by transcription of the compensatory sequence is as set forth in SEQ ID NO: 29; 13) the small RNA molecule has a sequence as set forth in SEQ ID NO: 30, and an RNA sequence produced by transcription of the compensatory sequence is as set forth in SEQ ID NO: 31; 14) the small RNA molecule has a sequence as set forth in SEQ ID NO: 32, and an RNA sequence produced by transcription of the compensatory sequence is as set forth in SEQ ID NO: 33; 15) the small RNA molecule has a sequence as set forth in SEQ ID NO: 34, and an RNA sequence produced by transcription of the compensatory sequence is as set forth in SEQ ID NO: 35; 16) the small RNA molecule has a sequence as set forth in SEQ ID NO: 36, and an RNA sequence produced by transcription of the compensatory sequence is as set forth in SEQ ID NO: 37; 17) the small RNA molecule has a sequence as set forth in SEQ ID NO: 38, and an RNA sequence produced by transcription of the compensatory sequence is as set forth in SEQ ID NO: 39; 18) the small RNA molecule has a sequence as set forth in SEQ ID NO: 40, and an RNA sequence produced by transcription of the compensatory sequence is as set forth in SEQ ID NO: 41; 19) the small RNA molecule has a sequence as set forth in SEQ ID NO: 42, and an RNA sequence produced by transcription of the compensatory sequence is as set forth in SEQ ID NO: 43; 20) the small RNA molecule has a sequence as set forth in SEQ ID NO: 44, and an RNA sequence produced by transcription of the compensatory sequence is as set forth in SEQ ID NO: 45; 21) the small RNA molecule has a sequence as set forth in SEQ ID NO: 46, and an RNA sequence produced by transcription of the compensatory sequence is as set forth in SEQ ID NO: 47; 22) the small RNA molecule has a sequence as set forth in SEQ ID NO: 48, and an RNA sequence produced by transcription of the compensatory sequence is as set forth in SEQ ID NO: 49; or 23) the small RNA molecule has a sequence as set forth in SEQ ID NO: 50, and an RNA sequence produced by transcription of the compensatory sequence is as set forth in SEQ ID NO: 51.

In another aspect, provided herein is an expression vector, comprising the above-described nucleic acid molecule.

In some embodiments, the expression vector further comprises a promoter sequence operably linked to the nucleic acid molecule.

In some embodiments, the expression vector is selected from a plasmid vector, an adeno-associated viral vector, an adenoviral vector, a retroviral vector, and a lentiviral vector.

In another aspect, provided herein is a host cell, comprising the above-described nucleic acid molecule or expression vector.

In another aspect, provided herein is a method for producing a small RNA molecule, comprising culturing the above-described host cell and optionally isolating the small RNA molecule.

In another aspect, provided herein is a method for producing an exosome comprising a small RNA molecule, comprising culturing the above-described host cell and isolating the exosome from a culture supernatant.

In another aspect, provided herein is the use of the above-described nucleic acid molecule, expression vector, or host cell in the preparation of a small RNA molecule.

In another aspect, provided herein is a pharmaceutical composition comprising: 1) the above-described expression vector or host cell; and 2) a pharmaceutically acceptable carrier.

In another aspect, provided herein is the use of the above-described expression vector or host cell in the preparation of a medicament for treating a disease, wherein the disease is related to an abnormal expression or mutation of a gene.

In another aspect, provided herein is a method for treating a disease in a subject, comprising administering to the subject a therapeutically effective amount of the above-described expression vector or host cell, wherein the disease is related to an abnormal expression or mutation of a gene.

When the nucleic acid molecule and expression vector provided herein are used for expression of a target small molecule RNA, the expression quantity of the target small molecule RNA in a host cell and the expression quantity of the target small molecule RNA in exosomes secreted by the host cell can be increased.

### Brief Description of the Drawings

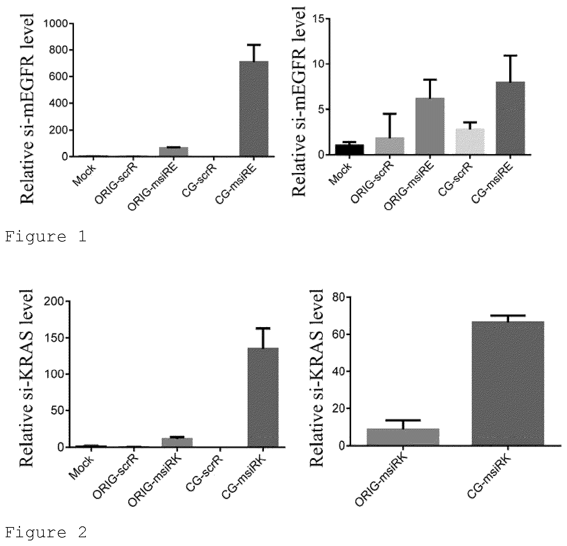

FIG. 1 shows that the CG-miR-155 backbone promotes the expression of the target nucleic acid si-mEGFR in cells and exosomes in some examples. Intracellular and extracellular expressions of si-mEGFR (msiRE) by the original backbone system (original) and CG-miR-155 backbone system are involved. The left panel shows relative levels of siRNA in 293FT cells. The right panel shows relative levels of siRNA secreted in exosomes. Mock represents a 293FT untreated group. ScrR represents si-scramble (negative control of siRNA) expressed by the miR-155 backbone.
FIG. 2 shows that the CG-miR-155 backbone promotes the expression of the target nucleic acid si-mKRAS in cells and exosomes in some examples. Intracellular and extracellular expressions of si-mKRAS (msiRK) by the original backbone system (ORIG) and CG-miR-155 backbone system are involved. The left panel shows relative levels of siRNA in 293FT cells. The right panel shows relative levels of siRNA secreted in exosomes. Mock represents a 293FT untreated group. ScrR represents si-scramble expressed by the miR-155 backbone.

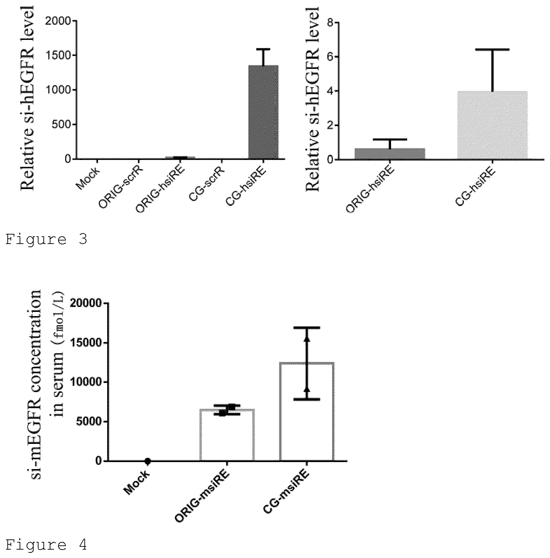

FIG. 3 shows that the CG-miR-155 backbone promotes the expression of the target nucleic acid si-hEGFR in cells and exosomes in some examples. Intracellular and extracellular expressions of si-hEGFR (hsiRE) by the original backbone system (ORIG) and CG-miR-155 backbone system are involved. The left panel shows relative levels of siRNA in 293FT cells. The right panel shows relative levels of siRNA secreted in exosomes. Mock represents a 293FT untreated group. ScrR represents si-scramble expressed by the miR-155 backbone.
FIG. 4 shows that the CG-miR-155 backbone promotes the expression of the target nucleic acid *in vivo.* Serum contents of si-mEGFR (msiRE) produced by the original backbone system (ORIG) and CG-miR-155 backbone system are involved. Mock represents a mouse serum from an untreated group.

### Detailed Description of Embodiments

Unless otherwise stated, all technical and scientific terms used herein have the meanings commonly understood by those of ordinary skill in the art.

The term "or" refers to an individual element of the listed optional elements, unless the context explicitly indicates otherwise. The term "and/or" refers to any one of, any two of, any three or more of, or all of the listed optional elements.

The term "contain" or "comprise" refers to the inclusion of the stated elements, integers, or steps, but not the exclusion of any other elements, integers, or steps. When the term "contain" or "comprise" is used herein, unless otherwise indicated, the situations where the subject matter consists of the described elements, integers, or steps are also encompassed. For example, when reference is made to a nucleic acid molecule "comprising" several particular sequences, it is also intended to encompass the nucleic acid molecule that consists of the particular sequences.

The term "about" generally refers to a variation within a range of 10% above or below a specified value, for example 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

The terms "nucleic acid molecule", "nucleic acid", and "polynucleotide" are used interchangeably herein and refer to a polymer of nucleotides. Such polymers of nucleotides may contain natural and/or unnatural nucleotides and include, but are not limited to, DNA, RNA, and PNA. A "nucleic acid sequence" refers to a linear sequence of nucleotides contained in a nucleic acid molecule or polynucleotide, typically presented in a 5' to 3' direction. An "isolated nucleic acid molecule" refers to a nucleic acid molecule that has been removed from its natural environment (e.g., the intracellular environment) and is substantially free of one or more substances that are normally associated with it in nature, such as proteins, nucleic acids, lipids, carbohydrates or cell membranes, or that is an artificially prepared (e.g., artificially synthesized) nucleic acid molecule. For DNA molecules, due to double-strand complementation, when the sequence of one strand is mentioned herein, those skilled in the art should recognize that its complementary strand or a double-stranded DNA molecule comprising its complementary strand has also been mentioned.

As used herein, the term "small RNA molecule" refers to an RNA molecule having a length of less than about 100 bases, for example, about 10-50 bases, or 10-30 bases. The small RNA molecule may be single-stranded or double-stranded, and includes, but is not limited to, siRNA, miRNA, hpRNA, and piRNA. In some cases, the length of the small RNA molecule may not be limited as described above, and mRNA molecules, for example, may be included.

The term "siRNA" refers to a small RNA molecule that can induce the degradation of its target RNA molecule through the phenomenon of RNA interference (RNAi). In cells, siRNA may be generated from longer double-stranded RNA molecules by cleavage with a nuclease known as Dicer. The Dicer nuclease cleaves a double-stranded RNA molecule to generate a shorter double-stranded siRNA molecule. The double-stranded siRNA molecule binds to a complex known as pre-RISC, and the Argonaute protein in the complex of the pre-RISC can cleave one strand of the siRNA molecule (referred to as the passenger strand). The cleavage product dissociates itself from the complex, and the other strand of the siRNA molecule (referred to as the guide strand) forms with the complex an RNA-induced silencing complex (RISC). The guide strand in the RISC can then bind to a target RNA molecule that contains a sequence complementary to the guide strand and, with the aid of the Argonaute protein in RISC, the target molecule is cleaved at specific sites, resulting in degradation of the target molecule. The target molecule may be, for example, viral RNA, transposon transcripts, and mRNA. siRNA molecules are typically completely complementary to their target sequences, which distinguishes them from miRNAs. RNAi induced by siRNA molecules is believed to be a defence mechanism by which organisms prevent viral replication *in vivo* or prevent transposon movement within the genome.

"miRNA" refers to a small RNA molecule that can lead to the degradation of its target RNA molecule (typically an mRNA molecule) or interfere with the translation of the mRNA molecule through the phenomenon of RNA interference (RNAi). In cells, miRNA molecules are typically products expressed by the cellular genome, for example, miRNA-155. In the nucleus, a transcribed single-stranded RNA molecule known as pri-miRNA forms a stem-loop structure with free ends by virtue of the reverse complementarity of some internal sequences within the molecule. The Drosha enzyme cleaves the double-stranded region at the stem to generate a smaller stem-loop RNA molecule, referred to as pre-miRNA. After the pre-miRNA is transferred from the nucleus to the cytoplasm, the Dicer enzyme cleaves the double-stranded region at the stem near the loop, generating a double-stranded miRNA molecule. Subsequently, an RISC is formed through a mechanism similar to that of siRNA processing, and the effect of RNAi is exerted. The target RNA molecule of miRNA is typically an intracellular mRNA, and the target sequence thereof is located in the 3' untranslated region (3'UTR) of the mRNA molecule. The RNA interference effect can be achieved by promoting degradation of the target mRNA or inhibiting its translation. Relatively speaking, when the miRNA molecule is completely complementary to the target sequence in its target mRNA molecule, it tends to promote the degradation of the target mRNA. RNAi induced by siRNA molecules is considered a way of regulating gene expression within cells. Different from the way siRNA functions, miRNA molecules often do not exhibit complete complementarity to their target molecules; for example, pairing may involve only several (e.g., six or seven) nucleotides at the 5' end of the guide strand (known as the "seed region") and a few nucleotides at other positions.

The length of siRNA or miRNA is typically about 10-30 nucleotides, more preferably 20-24 nucleotides (e.g., 20-23 nucleotides). When present in a double-stranded form, siRNA or miRNA may comprise short overhangs at each end. Preferably, the overhang at the 3' end has a length of 1-6 nucleotides.

Although the natural formation mechanisms of siRNA and miRNA in cells differ, their modes of action are similar in that they both can silence expression of target genes. Accordingly, small RNA molecules introduced into cells artificially or expressed via vectors for gene silencing purposes are collectively referred to herein as "siRNA molecules", that is, unless otherwise specified, no distinction is made between siRNA and miRNA.

"shRNA" refers to a single-stranded RNA molecule having a stem-loop or hairpin structure. These single-stranded RNA molecules have continuous complementary bases that can fold back on themselves, allowing the complementary base pairs to meet and pair via hydrogen bonding to form the stem of the stem-loop structure, while the sequence between the complementary sequences forms the loop of the stem-loop structure. As described above, shRNA may serve as a precursor molecule of miRNA molecules.

"piRNA" refers to a small RNA molecule capable of binding to a PIWI protein, an analogue of the Argonaute protein, and is typically 24-31 nt in length. piRNAs are mainly found in germ cells and stem cells of mammals, and regulate gene silencing through the formation of piRNA complexes (piRCs) in association with PIWI protein.

"miRNA backbone" and "miR backbone" are used interchangeably herein, and refer to a nucleic acid molecule or nucleotide fragment that can be used to express and produce small RNA molecules (such as siRNA or miRNA molecules) in cells. An miRNA backbone may include a coding sequence for a small RNA molecule, a 5' flanking sequence located in the 5' direction of the coding sequence, a 3' flanking sequence located in the 3' direction of the coding sequence, and a Loop sequence, (the transcription product of the Loop sequence corresponds to the Loop sequence of the stem-loop RNA molecule generated after transcription of the miRNA backbone), wherein the Loop sequence may be located between the coding sequence and the 3' flanking sequence, or located between the coding sequence and a 5' flanking sequence. Accordingly, in some embodiments, the miRNA backbone comprises in a 5' to 3' direction: a 5' flanking sequence - a coding sequence for a small RNA molecule - a Loop sequence - a 3' flanking sequence. In other embodiments, the miRNA backbone comprises in a 5' to 3' direction: a 5' flanking sequence - a Loop sequence - a coding sequence for a small RNA molecule - a 3' flanking sequence. In some embodiments, the miRNA backbone further comprises a compensatory sequence (or known as a passenger strand), which is at least partially complementary (e.g., at least 80%, at least 85%, or at least 90% complementary) to the coding sequence for the small molecule RNA, such that the transcription product thereof forms the stem together with the transcription product of the coding sequence in the stem-loop RNA molecule generated after transcription of the miRNA backbone. Accordingly, in some embodiments, the miRNA backbone comprises in a 5' to 3' direction: a 5' flanking sequence - a coding sequence for a small RNA molecule - a Loop sequence - a compensatory sequence - a 3' flanking sequence. In other embodiments, the miRNA backbone comprises in a 5' to 3' direction: a 5' flanking sequence - a compensatory sequence - a Loop sequence - a coding sequence for a small RNA molecule - a 3' flanking sequence. In some embodiments, the compensatory sequence has a length the same as that of the coding sequence. In some embodiments, the compensatory sequence has a length different from that of the coding sequence, e.g., 1-5 nucleotides longer or 1-5 nucleotides shorter than the coding sequence. In some embodiments, the expression product of the compensatory sequence given herein is degraded as a passenger strand. The coding sequence (and the compensatory sequence) for a small RNA molecule can be designed on the basis of the sequence of the target RNA molecule, depending on the sequence of the RNA molecule targeted. In some embodiments, the miRNA backbone provided herein is obtained by modification of the murine genomic sequence expressing miR-155. In some embodiments, the murine miR-155 backbone used has the following sequence (hereinafter referred to as the "original miR-155 backbone sequence" or original backbone system): *GGATCCTGGAGGCTTGCTGAAGGCTGTATGCTGAATTCG* (N)₁₀₋₃₀GTTTTGGCCACTGACTGAC(N)₁₀₋₃₀**CACCGGTCAGGACACAAGGC CTGTTACTAGCACTCACATGGAACAAATGGCCCAGATCTGGCCGCA CTCGAG** (SEQ ID NO: 5), wherein the two (N)₁₀₋₃₀ respectively correspond to the mature miRNA coding sequence and the compensatory sequence for the miR-155 molecule. The sequence shown in italics in this original backbone sequence corresponds to the above-described 5' flanking sequence, the underlined sequence corresponds to the above-described Loop sequence, and the sequence shown in bold corresponds to the above-described 3' flanking sequence. The inventors have surprisingly found that in the original backbone sequence, deleting several (e.g., 5-7) nucleotide bases at the 3' end of the 5' flanking sequence, and deleting several (e.g., 6-8) nucleotide bases at the 5' end of the 3' flanking sequence yield an miRNA backbone (referred to as the CG-miR-155 backbone) that can increase the expression quantity of the miR-155 molecule. Furthermore, when the coding sequence and compensatory sequence for the miR-155 molecule are replaced with the coding sequence and compensatory sequence for an miRNA or siRNA molecule of different sequences, the expression quantity of the miRNA molecule can also be enhanced. The inventors have further found that the increase in expression quantity is observed not only within cells, but also in exosomes secreted by the cells.

In some embodiments, the 5' flanking sequence in the modified miR-155 backbone provided herein comprises a sequence as set forth in SEQ ID NO: 1, a sequence having at least 80% (e.g., at least 85%, 90%, or 95%) sequence identity to the sequence as set forth in SEQ ID NO: 1, or a sequence comprising up to 5 base differences (including insertions, deletions, or substitutions) compared to the sequence as set forth in SEQ ID NO: 1.

In some embodiments, the 3' flanking sequence in the modified miR-155 backbone provided herein comprises a sequence as set forth in SEQ ID NO: 2, a sequence having at least 80% (e.g., at least 85%, 90%, or 95%) sequence identity to the sequence as set forth in SEQ ID NO: 2, or a sequence comprising up to 5 base differences (including insertions, deletions, or substitutions) compared to the sequence as set forth in SEQ ID NO: 2.

In some embodiments, the Loop sequence in the modified miR-155 backbone provided herein comprises a sequence as set forth in SEQ ID NO: 3, a sequence having at least 80% (e.g., at least 85%, 90%, or 95%) sequence identity to the sequence as set forth in SEQ ID NO: 3, or a sequence comprising up to 5 base differences (including insertions, deletions, or substitutions) compared to the sequence as set forth in SEQ ID NO: 3.

In a preferred embodiment, the modified miR-155 backbone provided herein (hereinafter referred to as the CG-miR-155 backbone) comprises the following sequence: GGATCCTGGAGGCTTGCTGAAGGCTGTATGCTG(N)₁₀₋₃₀GTTTT GGCCACTGACTGAC(N)₁₀₋₃₀CAGGACACAAGGCCTGTTACTAGCACTCAC ATGGAACAAATGGCCCAGATCTGGCCGCACTCGAG (SEQ ID NO: 4), wherein the two (N)₁₀₋₃₀ correspond to the coding sequence for the target small RNA molecule (such as siRNA or miRNA) and the compensatory sequence, the two having the same or different lengths. Compared to the original miR-155 backbone sequence, the modified miR-155 backbone does not contain consecutive "AATTCG" bases at the 3' end of the 5' flanking sequence and does not contain consecutive "CACCGGT" bases at the 5' end of the 3' flanking sequence.

An "expression vector" herein refers to a nucleic acid construct used to express (transcribe) RNA that can be used to produce a small molecule RNA. The expression vector provided herein may comprise the CG-miR-155 backbone described above, a promoter operably linked thereto, and suitable transcription initiation and termination sequences. A "promoter" is a stretch of DNA sequence where a RNA polymerase recognizes, binds and initiates transcription, and contains a conserved sequence required for specific binding to the RNA polymerase and initiation of transcription. Most promoters are located upstream of transcription initiation sites. The promoter itself is not transcribed. The promoters suitable for RNA polymerase II (e.g., CMV and EF1A) or for RNA polymerase III (e.g., U6 and H1) can be used in the expression vector provided herein. In addition, the expression vector has the ability to replicate within the host, which is usually conferred by the origin of replication, and/or has a selection gene that helps to identify transformants. In general, expression vectors used in recombinant DNA technology are often in the form of "plasmids", which refer to circular double-stranded DNA loops. Obviously, vectors derived from viruses such as retroviruses and adenoviruses can also be used.

"Operably linked" means that a regulatory sequence is linked to its regulatory object in such a way that the regulatory sequence can act on its regulatory object. For example, a promoter being "operably linked" to a target gene means that the promoter can drive the transcription of the target gene from the exact start site.

Exosomes are small membrane vesicles that can be secreted by a variety of cells, having a lipid bilayer membrane structure and a diameter of approximately 10-300 nm (other sizes are also possible). Exosomes may contain proteins, lipids, and nucleic acids (such as small RNA molecules) specific to the cells from which they are secreted, and can serve as mediators of intercellular information and material exchange. Exosomes play an important role in many physiological and pathological processes, such as antigen presentation in immunity, tumour growth and metastasis, and repair of tissue damage. Exosomes secreted by different cells possess different components and functions, and may serve as biomarkers for disease diagnosis. Due to their lipid bilayer membrane structure, exosomes can effectively protect the substances encapsulated therein and are capable of circulating between cells or tissues; thus, exosomes can be used as tools for delivery and drug administration.

A "host cell" refers to a cell that can be or has been a recipient of an expression vector or an isolated polynucleotide. The host cell can be a prokaryotic cell or a eukaryotic cell. Exemplary eukaryotic cells include mammalian cells, such as primate or non-primate cells; fungal cells, such as yeast; plant cells; and insect cells. Non-limiting exemplary mammalian cells include, but are not limited to, NSO cells, 293, and CHO cells, and derived cells thereof, such as 293-6E, CHO-DG44, CHO-K1, CHO-S, and CHO-DS cells. Host cells include the offspring of a single host cell, and the offspring may not be exactly the same as the original parent cell (in terms of morphology or genomic DNA complementarity) due to natural, accidental or intentional mutation. Host cells also include cells transfected with nucleic acid molecules or expression vectors provided herein *in vivo.*

The term "pharmaceutically acceptable carrier" as used in reference to pharmaceutical compositions refers to materials such as solid or liquid diluents, fillers, antioxidants, and stabilizers with which can be safely administered, and which are suitable for administration to a subject without undue adverse side effects, while maintaining the activity of the drug or active agent contained therein. According to the route of administration, various carriers well known in the art may be used, including, but not limited to, sugars, starches, cellulose and derivatives thereof, maltose, gelatine, talc, calcium sulphate, vegetable oils, synthetic oils, polyols, alginic acids, phosphate buffer solutions, emulsifiers, isotonic saline and/or pyrogen-free water. The pharmaceutical composition provided herein can be formulated into a clinically acceptable dosage form such as a powder and an injection. The pharmaceutical composition of the present invention may be administered to a subject via any suitable route, such as oral administration, intravenous infusion, intramuscular injection, subcutaneous injection, subperitoneal administration, rectal administration, sublingual administration, or via inhalation, transdermal delivery and other routes. In a preferred embodiment, the pharmaceutical composition is formulated in such a way that is suitable for intravenous or intramuscular administration in accordance with conventional procedures. Typically, pharmaceutical compositions for intravenous or intramuscular administration are solutions in a sterile isotonic aqueous buffer.

"Subject" as used herein refers to animals, such as mammals, including, but not limited to, humans, rodents, simians, felines, canines, equines, bovines, porcines, ovines, caprines, mammalian laboratory animals, mammalian farm animals, mammalian sport animals, and mammalian pets. The subject may be male or female and may be any subject of appropriate age, including infant, juvenile, adolescent, adult, and geriatric subjects. In some examples, a subject refers to an individual in need of treatment for a disease or condition. In some examples, the subject receiving the treatment may be a patient who is suffering from, or at risk of developing, a condition associated with the treatment. In a particular example, the subject is a human, such as a human patient. The term is often used interchangeably with "patient", "detection object", "treatment object", etc.

When referring to nucleotide sequences, the term "sequence identity" (also known as "Sequence Identity") refers to the degree of identity between two nucleotide sequences (e.g., a query sequence and a reference sequence), generally expressed as a percentage. Generally, before calculating the percentage of identity between two nucleotide sequences, sequence alignment is performed first and gaps (if any) are introduced. If bases in the two sequences are the same at a certain alignment position, the two sequences are considered to be identical or matched at that position. If the bases in the two sequences are different, the two sequences are considered to be non-identical or mismatched at that position. In some algorithms, the number of matched positions is divided by the total number of positions in the alignment window to obtain sequence identity. In other algorithms, the number and/or the length of gaps is also taken into account. For the purpose of the present invention, the publicly available alignment software BLAST (available at website: ncbi.nlm.nih.gov) can be employed to obtain the optimal sequence alignment and calculate the sequence identity between two nucleotide sequences using default settings. In some embodiments, "at least 90% sequence identity" as described herein includes, but is not limited to: at least 95%, at least 98%, at least 99%, or even 100% sequence identity.

In one aspect, the present invention provides an miR backbone sequence comprising a 5' flanking sequence, a Loop sequence, and a 3' flanking sequence, wherein a coding sequence for a small molecule RNA targeting a target nucleic acid, such as a target gene, may be inserted between the 5' flanking sequence and the Loop sequence. The miR backbone sequence can increase the expression level of the small molecule RNA in cells and exosomes.

The 5' flanking sequence comprises a sequence as set forth in SEQ ID NO: 1 or a sequence having at least 80% homology thereto. Preferably, consecutive "AATTCG" bases are not contained at the 3' end of the 5' flanking sequence. Preferably, the 5' flanking sequence comprises a sequence as set forth in SEQ ID NO: 1 or a variant sequence having no more than 5 base differences therefrom.

The 3' flanking sequence comprises a sequence as set forth in SEQ ID NO: 2 or a sequence having at least 80% homology thereto. Preferably, consecutive "CACCGGT" bases are not contained at the 5' end of the 3' flanking sequence. Preferably, the 3' flanking sequence comprises a sequence as set forth in SEQ ID NO: 2 or a variant sequence having no more than 5 base differences therefrom.

The Loop sequence comprises a sequence as set forth in SEQ ID NO: 3 or a sequence having at least 80% homology thereto.

In some embodiments, the miR backbone is a CG-miR-155 backbone.

In another aspect, the present invention provides an expression vector comprising the miR backbone, coding sequence for the small molecule RNA, and compensatory sequence described above. The compensatory sequence is reversely complementary to at least 80% of the bases of the coding sequence for the small molecule RNA and, after transcription, cooperates with the transcription product of the Loop sequence to form a stem-loop structure. In some specific embodiments, the compensatory sequence is reversely complementary to 90% of the bases of the coding sequence for the small molecule RNA.

The coding sequence product for the small molecule RNA comprises at least one siRNA, miRNA, shRNA, or mRNA. Preferably, the small molecule RNA comprises 10-30 nucleotides. Preferably, the small molecule RNA is an siRNA that specifically silences the expression of the target gene.

In some specific embodiments, the miR backbone sequence comprises a 5' flanking sequence - an siRNA-Loop sequence - a compensatory sequence - a 3' flanking sequence from the 5' end to the 3' end.

The expression vector may also contain a promoter sequence.

The expression vector further comprises a targeting sequence that targets a particular tissue or organ, or its encoded product (polypeptide) may target a particular tissue or organ.

The target gene is a disease-related gene that is highly expressed in diseased tissues, the disease including but not limited to neurological diseases, ophthalmic diseases (VEGF, VEGFR, ANG1, and ANG2), infectious disease, scar hyperplasia, chronic disease, cancer, or other indications.

The target genes include, but are not limited to, ataxin-2, SLN, SOD1, ATXN3, C9ORF72, HTT, VEGF, VEGFR, ANG1, ANG2, SARS-CoV-2, HBV, HCV, HIV, HPV, HSV, COX-2, TGF-beta, GBAI, PCSK9, LDL, KRAS, NKRAS, HRAS, EGFR, ATL, MET, PIK3CA, SHP1, SOX2, PTEN, TP53, Yap1, HIF1a, CDK1, CDK4, CDK6, NRF2, beta-Catenin, STAT3, Aurora A, CDH17, CXCR4, IDO1, CSF-1, NF-kB, NOTCH, SOX9, PD-L1, PD1, CTLA4, LAG3, TIGIT, COX-2, TGF-beta, VEGF, VEFGR, BRAF, and other genes.

Preferably, the target gene is a cancer-related gene, which is highly expressed in cancer tissues. The cancer-related genes include, but are not limited to, KRAS and/or EGFR.

In some embodiments, the sequence of the small RNA molecule and the corresponding passenger strand sequence are shown in Table 1.

In another aspect, the present invention provides a pharmaceutical composition comprising the above-described expression vector and an acceptable pharmaceutical carrier.

In a further aspect, the present invention provides the use of the miR backbone or expression vector described above for the treatment of a disease.

In yet another aspect, the present invention provides a method for treating a disease, comprising injecting an effective amount of the expression vector or pharmaceutical composition described above into a subject.

The miR backbone or expression vector provided in the present invention can significantly increase the expression quantity of small RNA molecules in cells and exosomes, while also increasing the expression quantity *in vivo.*

In some embodiments, the CG-miR-155 backbone is an optimized miR-155 backbone sequence, specifically comprising an optimization of the 5'-end and 3'-end flanking sequences. After optimization, the 5' flanking sequence comprises a sequence as set forth in SEQ ID NO: 1 or a sequence having at least 80% homology thereto. Preferably, consecutive "AATTCG" bases are not contained at the 3' end of the 5' flanking sequence. The 3' flanking sequence comprises a sequence as set forth in SEQ ID NO: 2 or a sequence having at least 80% homology thereto. Preferably, consecutive "CACCGGT" bases are not contained at the 5' end of the 3' flanking sequence. Preferably, the Loop sequence comprises a sequence as set forth in SEQ ID NO: 3 or a sequence having at least 80% homology thereto. Preferably, the coding sequence for the target siRNA is inserted between the 5' flanking sequence and the Loop sequence or inserted between the Loop sequence and the 3' flanking sequence. Preferably, the compensatory sequence corresponding to the coding sequence for the target siRNA is inserted between the Loop sequence and the 3' flanking sequence or inserted between the 5' flanking sequence and the Loop sequence. Preferably, the compensatory sequence comprises a sequence that is completely complementary to or has at least 80% homology to the siRNA coding sequence. Preferably, the compensatory sequence, on the basis of being completely complementary to the siRNA coding sequence, has 2-3 consecutive bases deleted in bases 7-12 from the 5' end to the 3' end. Preferably, the compensatory sequence, on the basis of being completely complementary to the siRNA sequence, has bases 9 and 10 deleted from the 5' end to the 3' end.

The technical solutions of the present invention are further illustrated in detail by the examples and in conjunction with the accompanying drawings. Unless otherwise specified, the methods and materials in the examples described below are conventional products that can be purchased from the market. Those skilled in the art of the present invention would understand that the methods and materials described below are exemplary only and should not be considered as limiting the scope of the present invention.

### Example 1: CG-miR-155 backbone promoting expression of target nucleic acid si-mEGFR in cells and exosomes

The small RNA molecule si-mEGFR and the compensatory sequence thereof, as set forth in SEQ ID NOs: 6 and 7, were loaded onto the CG-miR-155 backbone (SEQ ID NO: 4), and plasmids were directly synthesized. Plasmids containing a promoter sequence were provided by Genscript Biotech Co., Ltd., with the CMV promoter being an optional choice. 5 µg of plasmids containing the corresponding sequences were transfected into 1E6 cells/well 293FT cells using the PEI transfection method. After 48 hours of transfection, the cells and the supernatant were collected. Exosomes were isolated and collected from the culture supernatant using the Total Exosome Isolation Reagent (from cell culture media) (Invitrogen, 4478360) according to the instructions. For lysis of cells and exosomes, 1 ml of RNAiso for Small RNA (TAKARA, 9753A) was added to the sample and mixed uniformly by vigorous shaking, and then the mixture was left to stand at room temperature for 5 minutes. Subsequently, 0.2 ml of trichloromethane (Sinopharm, 67-66-3) was added and mixed uniformly by vigorous shaking, and then the mixture was left to stand at room temperature for 5 minutes. After phase separation of the mixture, the centrifuge tube was centrifuged at 14,000 g for 20 minutes at 4°C in a refrigerated centrifuge. The upper clear liquid was transferred to a new centrifuge tube, and an equal volume of isopropanol (Sinopharm, 67-63-0) was added and mixed uniformly by turning the tube upside down. Subsequently, the cell sample was left to stand at -20°C for 20 minutes, and the exosome sample was left to stand at -20°C overnight. The centrifuge tube after standing was centrifuged at 14,000 g for 20 minutes at 4°C in a refrigerated centrifuge. The supernatant was removed. The pellet was washed with 1 ml of 75% ethanol, and the centrifuge tube was centrifuged at 14,000 g for 10 minutes at 4°C in a refrigerated centrifuge. The supernatant was removed. After the liquid in the centrifuge tube was air-dried, the RNA pellet was dissolved in RNase-free water (yeasen, 10601ES76), and the RNA concentration was measured. Reverse transcription was performed using the Mir-X^{™} miRNA First Strand Synthesis Kit (TAKARA, 638315). By using the Mir-X^{™} miRNA qRT-PCR TB Green^{®} Kit (TAKARA, 638316), qPCR assays were performed on the internal reference gene (U6 for the intracellular internal reference gene and miR-16U6 for the internal reference gene in exosomes) and the target gene. The relative expression quantity of the target gene in the sample was calculated, as shown in FIG. 1.

### Example 2: CG-miR-155 backbone promoting expression of small RNA molecule si-hKRAS in cells and exosomes.

The small RNA molecule si-hKRAS and the compensatory sequence thereof, as shown in SEQ ID NOs: 30-51, were loaded onto the CG-miR-155 backbone, and plasmids were prepared (the plasmid synthesis was performed by Genscript Biotech Co., LTD.). 5 µg of plasmids containing the corresponding sequences were transfected into 1E6 cells/well 293FT cells using the PEI transfection method. After 48 hours of transfection, the cells and the supernatant were collected. Exosomes were isolated and collected from the culture supernatant using the Total Exosome Isolation Reagent (from cell culture media) (Invitrogen, 4478360) according to the instructions. For lysis of cells and exosomes, 1 ml of RNAiso for Small RNA (TAKARA, 9753A) was added to the sample and mixed uniformly by vigorous shaking, and then the mixture was left to stand at room temperature for 5 minutes. Subsequently, 0.2 ml of trichloromethane (Sinopharm, 67-66-3) was added and mixed uniformly by vigorous shaking, and then the mixture was left to stand at room temperature for 5 minutes. After phase separation of the mixture, the centrifuge tube was centrifuged at 14,000 g for 20 minutes at 4°C in a refrigerated centrifuge. The upper clear liquid was transferred to a new centrifuge tube, and an equal volume of isopropanol (Sinopharm, 67-63-0) was added and mixed uniformly by turning the tube upside down. Subsequently, the cell sample was left to stand at -20°C for 20 minutes, and the exosome sample was left to stand at -20°C overnight. The centrifuge tube after standing was centrifuged at 14,000 g for 20 minutes at 4°C in a refrigerated centrifuge. The supernatant was removed. The pellet was washed with 1 ml of 75% ethanol, and the centrifuge tube was centrifuged at 14,000 g for 10 minutes at 4°C in a refrigerated centrifuge. The supernatant was removed. After the liquid in the centrifuge tube was air-dried, the RNA pellet was dissolved in RNase-free water (yeasen, 10601ES76), and the RNA concentration was measured. Reverse transcription was performed using the Mir-X^{™} miRNA First Strand Synthesis Kit (TAKARA, 638315). By using the Mir-X^{™} miRNA qRT-PCR TB Green^{®} Kit (TAKARA, 638316), qPCR assays were performed on the internal reference gene and the target gene. The relative expression quantity of the target gene in the sample was calculated, as shown in FIG. 2.

### Example 3: CG-miR-155 backbone promoting expression of small RNA molecule si-hEGFR in cells and exosomes.

The small RNA molecule si-hEGFR and the compensatory sequence thereof, as shown in SEQ ID NOs: 8-29, were loaded onto the CG-miR-155 backbone, and plasmids were prepared (the plasmid synthesis was performed by Genscript Biotech Co., LTD.). 5 µg of plasmids containing the corresponding sequences were transfected into 1E6 cells/well 293FT cells using the PEI transfection method. After 48 hours of transfection, the cells and the supernatant were collected. Exosomes were isolated and collected from the culture supernatant using the Total Exosome Isolation Reagent (from cell culture media) (Invitrogen, 4478360) according to the instructions. For lysis of cells and exosomes, 1 ml of RNAiso for Small RNA (TAKARA, 9753A) was added to the sample and mixed uniformly by vigorous shaking, and then the mixture was left to stand at room temperature for 5 minutes. Subsequently, 0.2 ml of trichloromethane (Sinopharm, 67-66-3) was added and mixed uniformly by vigorous shaking, and then the mixture was left to stand at room temperature for 5 minutes. After phase separation of the mixture, the centrifuge tube was centrifuged at 14,000 g for 20 minutes at 4°C in a refrigerated centrifuge. The upper clear liquid was transferred to a new centrifuge tube, and an equal volume of isopropanol (Sinopharm, 67-63-0) was added and mixed uniformly by turning the tube upside down. Subsequently, the cell sample was left to stand at -20°C for 20 minutes, and the exosome sample was left to stand at -20°C overnight. The centrifuge tube after standing was centrifuged at 14,000 g for 20 minutes at 4°C in a refrigerated centrifuge. The supernatant was removed. The pellet was washed with 1 ml of 75% ethanol, and the centrifuge tube was centrifuged at 14,000 g for 10 minutes at 4°C in a refrigerated centrifuge. The supernatant was removed. After the liquid in the centrifuge tube was air-dried, the RNA pellet was dissolved in RNase-free water (yeasen, 10601ES76), and the RNA concentration was measured. Reverse transcription was performed using the Mir-X^{™} miRNA First Strand Synthesis Kit (TAKARA, 638315). By using the Mir-X^{™} qRT-PCR TB Green^{®} Kit (TAKARA, 638316), qPCR assays were performed on the internal reference gene and the target gene. The relative expression quantity of the target gene in the sample was calculated, as shown in FIG. 3.

### Example 4: CG-miR-155 backbone promoting expression of small RNA molecules in vivo.

The small RNA molecule si-mEGFR was loaded onto the CG-miR-155 backbone, and plasmids were prepared. C57 mice were injected with the plasmids via the tail vein at a dose of 80 mg/ml and an injection volume of 2 ml, which was completed within 7 s. Serum samples were collected 24 hours after injection. For lysis of cells and exosomes, 1 ml of RNAiso for Small RNA (TAKARA, 9753A) was added to the serum sample and mixed uniformly by vigorous shaking, and then the mixture was left to stand at room temperature for 5 minutes. Subsequently, 0.2 ml of trichloromethane (Sinopharm, 67-66-3) was added and mixed uniformly by vigorous shaking, and then the mixture was left to stand at room temperature for 5 minutes. After phase separation of the mixture, the centrifuge tube was centrifuged at 14,000 g for 20 minutes at 4°C in a refrigerated centrifuge. The upper clear liquid was transferred to a new centrifuge tube, and an equal volume of isopropanol (Sinopharm, 67-63-0) was added and mixed uniformly by turning the tube upside down. Subsequently, the centrifuge tube was left to stand at -20°C overnight. The centrifuge tube after standing was centrifuged at 14,000 g for 20 minutes at 4°C in a refrigerated centrifuge. The supernatant was removed. The pellet was washed with 1 ml of 75% ethanol, and the centrifuge tube was centrifuged at 14,000 g for 10 minutes at 4°C in a refrigerated centrifuge. The supernatant was removed. After the liquid in the centrifuge tube was air-dried, the RNA pellet was dissolved in RNase-free water (yeasen, 10601ES76), and the RNA concentration was measured. Reverse transcription was performed using RT primers from the TaqMan MicroRNA Reverse Transcription Kit (Applied Biosystems, 4366597) and CustomTaqManSmallRNAAssay (Applied Biosystems, 4398987) according to the instructions. qPCR assays were performed using probe-primer mixtures from TagMan^{™} Universal Master Mix II, with UNG (Applied Biosystems, 4440038) and CustomTaqManSmallRNAAssay (Applied Biosystems, 4398987). The single-stranded siRNA was used as a standard. After reverse transcription, gradient dilution was performed. qPCR detection was performed on the samples to obtain the relative quantity value of siRNA in the samples, and the siRNA content in serum was calculated. The results are shown in FIG. 4.

As can be seen from the above results, compared to the original backbone system, the miR backbone provided herein can significantly increase the expression quantity of a target small RNA molecule by cells, whether in the cells or in exosomes, or in animals.

The embodiments of the present invention are not limited to the above examples, and various modifications and improvements in forms and details may be made to the present invention by those of ordinary skill in the art without departing from the spirit and scope of the present invention, and these modifications and improvements are considered to fall within the scope of protection of the present invention.

The nucleotide sequences referred to herein are as shown in Table 1 below.

**Table 1. Nucleotide sequence**

| Sequence description | Sequence |
|---|---|
| 5' flanking sequence | GGATCCTGGAGGCTTGCTGAAGGCTGTATGCTG (SEQ ID NO: 1) |
| 3' flanking sequence | CAGGACACAAGGCCTGTTACTAGCACTCACATGGAACAAAT GGCCCAGATCTGGCCGCACTCGAG (SEQ ID NO: 2) |
| Loop sequence | GTTTTGGCCACTGACTGAC (SEQ ID NO: 3) |
| CG-miR-155 | GGATCCTGGAGGCTTGCTGAAGGCTGTATGCTG(N)10- 30GTTTTGGCCACTGACTGAC(N)10- 30CAGGACACAAGGCCTGTTACTAGCACTCACATGGAACAAA TGGCCCAGATCTGGCCGCACTCGAG (SEQ ID NO: 4) |
| MIR-155 | GGATCCTGGAGGCTTGCTGAAGGCTGTATGCTGAATTCG(N)1 0-30GTTTTGGCCACTGACTGAC(N)10- 30CACCGGTCAGGACACAAGGCCTGTTACTAGCACTCACATG GAACAAATGGCCCAGATCTGGCCGCACTCGAG (SEQ ID NO: 5) |
| si-mEGFR | Guide: UGUGGCUUCUCUUAACUCCU (SEQ ID NO: 6) |
| | Passenger: AGGAGUUAAGAAGCCACA (SEQ ID NO: 7) |
| si-hEGFR | Guide: UGUUGCUUCUCUUAAUUCCU (SEQ ID NO: 8) |
| | Passenger: AGGAAUUAAGAAGCAACA (SEQ ID NO: 9) |
| si-hEGFR | Guide: AAAUGAUCUUCAAAAGUGCCC (SEQ ID NO: 10) |
| | Passenger: GCACUUUUGGAUCAUUUUC (SEQ ID NO: 11) |
| si-hEGFR | Guide: AAAAUGAUCUUCAAAAGUGCC (SEQ ID NO: 12) |
| | Passenger: CACUUUUAGAUCAUUUUCU (SEQ ID NO: 13) |
| si-hEGFR | Guide: AGGUAAUUUCCAAAUUCCCAA (SEQ ID NO: 14) |
| | Passenger: GGGAAUUUGAAUUACCUAU (SEQ ID NO: 15) |
| si-hEGFR | Guide: UAGGUAAUUUCCAAAUUCCCA (SEQ ID NO: 16) |
| | Passenger: GGAAUUUGGAUUACCUAUG (SEQ ID NO: 17) |
| si-hEGFR | Guide: AUAGGUAAUUUCCAAAUUCCC (SEQ ID NO: 18) |
| | Passenger: GAAUUUGGAUUACCUAUGU (SEQ ID NO: 19) |
| si-hEGFR | Guide: UAAUUCCUCUGCACAUAGGUA (SEQ ID NO: 20) |
| | Passenger: CCUAUGUGCAGGAAUUAUG (SEQ ID NO: 21) |
| si-hEGFR | Guide: UUAAGAAGGAAAGAUCAUAAU (SEQ ID NO: 22) |
| | Passenger: UAUGAUCUCCUUCUUAAAG (SEQ ID NO: 23) |
| si-hEGFR | Guide: UUUAAGAAGGAAAGAUCAUAA (SEQ ID NO: 24) |
| | Passenger: AUGAUCUCUUCUUAAAGA (SEQ ID NO: 25) |
| si-hEGFR | Guide: UCUUUAAGAAGGAAAGAUCAU (SEQ ID NO: 26) |
| | Passenger: GAUCUUUCCUUAAAGACC (SEQ ID NO: 27) |
| si-hEGFR | Guide: GUUUUCCAAAGGAAUUCGCUC (SEQ ID NO: 28) |
| | Passenger: GCGAAUUUUGGAAAACCU (SEQ ID NO: 29) |
| si-hKRAS | Guide: GCAAAUACACAAAGAAAGCCC (SEQ ID NO: 30) |
| | Passenger: GGGCUUUCUGUGUAUUUGC (SEQ ID NO: 31) |
| si-hKRAS | Guide: UUUAUAUUCAGUCAUUUUCAG (SEQ ID NO: 32) |
| | Passenger: GAAAAUGGAAUAUAAACU (SEQ ID NO: 33) |
| si-hKRAS | Guide: UUCUGAAUUAGCUGUAUCGUC (SEQ ID NO: 34) |
| | Passenger: CGAUACAGAAUUCAGAAUC (SEQ ID NO: 35) |
| si-hKRAS | Guide: UAUUGUUGGAUCAUAUUCGUC (SEQ ID NO: 36) |
| | Passenger: CGAAUAUGCCAACAAUAGA (SEQ ID NO: 37) |
| si-hKRAS | Guide: UUAUUUAUGGCAAAUACACAA (SEQ ID NO: 38) |
| | Passenger: GUGUAUUUCAUAAAUAAUA (SEQ ID NO: 39) |
| si-hKRAS | Guide: UAUUAUUUAUGGCAAAUACAC (SEQ ID NO: 40) |
| | Passenger: GUAUUUGCUAAAUAAUACU (SEQ ID NO: 41) |
| si-hKRAS | Guide: AUAUCUUCAAAUGAUUUAGUA (SEQ ID NO: 42) |
| | Passenger: CUAAAUCAGAAGAUAUUC (SEQ ID NO: 43) |
| si-hKRAS | Guide: UAACUCUUUUAAUUUGUUCUC (SEQ ID NO: 44) |
| | Passenger: GAACAAAUUAAGAGUUAAG (SEQ ID NO: 45) |
| si-hKRAS | Guide: AUAACUUCUUGCUAAGUCCUG (SEQ ID NO: 46) |
| | Passenger: GGACUUAGCGAAGUUAUGG (SEQ ID NO: 47) |
| si-hKRAS | Guide: UUUCAAUCUGUAUUGUCGGAU (SEQ ID NO: 48) |
| | Passenger: CCGACAAUACAUUGAAAAA (SEQ ID NO: 49) |
| si-hKRAS | Guide: UUUUCAAUCUGUAUUGUCGGA (SEQ ID NO: 50) |
| | Passenger: CGACAAUACAUUGAAAAAA (SEQ ID NO: 51) |

## Claims

1. An isolated nucleic acid molecule, **characterized by** comprising in a 5'-3' direction:
1) a 5' flanking sequence comprising a sequence as set forth in SEQ ID NO: 1 or a sequence having at least 80% sequence identity thereto;
2) a Loop sequence comprising a sequence as set forth in SEQ ID NO: 3 or a sequence having at least 80% sequence identity thereto; and
3) a 3' flanking sequence comprising a sequence as set forth in SEQ ID NO: 2 or a sequence having at least 80% sequence identity thereto,
wherein the nucleic acid molecule further comprises a coding sequence for a small RNA molecule, and the coding sequence for the small RNA molecule is located between the 5' flanking sequence and the Loop sequence or between the Loop sequence and the 3' flanking sequence.

2. The nucleic acid molecule according to claim 1, **characterized in that** the 5' flanking sequence does not comprise the sequence AATTCG at the 3' end, and/or the 3' flanking sequence does not comprise the sequence CACCGGT at the 5' end.

3. The nucleic acid molecule according to claim 1 or 2, **characterized by** further comprising a compensatory sequence, wherein when the coding sequence for the small RNA molecule is located between the 5' flanking sequence and the Loop sequence, the compensatory sequence is located between the Loop sequence and the 3' flanking sequence; when the coding sequence for a small RNA molecule is located between the Loop sequence and the 3' flanking sequence, the compensatory sequence is located between the 5' flanking sequence and the Loop sequence, and the compensatory sequence is at least partially reversely complementary to the coding sequence for the small RNA molecule.

4. The nucleic acid molecule according to any one of claims 1-3, **characterized in that** the compensatory sequence is complementary to at least 80% of bases in the coding sequence for the small RNA molecule; preferably, compared with a sequence fully reversely complementary to the coding sequence for the small RNA molecule, the compensatory sequence lacks any 2-3 consecutive bases at positions 7-12 from the 5' end; more preferably, compared with a sequence fully reversely complementary to the coding sequence for the small RNA molecule, the compensatory sequence lacks bases at positions 9 and 10 from the 5' end.

5. The nucleic acid molecule according to any one of claims 1-4, **characterized in that** in a transcript RNA produced by transcription of the nucleic acid molecule, transcription products of the coding sequence for the small RNA molecule, the Loop sequence and the compensatory sequence are capable of forming a stem-loop structure, wherein the transcription product of the coding sequence for the small RNA molecule and the transcription product of the compensatory sequence form a stem of the stem-loop structure, and the transcription product of the Loop sequence forms a loop of the stem-loop structure.

6. The nucleic acid molecule according to any one of claims 1-5, **characterized in that** the transcript RNA is a pre-miRNA.

7. The nucleic acid molecule according to any one of claims 1-6, **characterized in that** the small RNA molecule is an miRNA or siRNA molecule, preferably 20-24 nt in length.

8. The nucleic acid molecule according to any one of claims 1-7, **characterized in that** the small RNA molecule targets a disease-associated gene expressed in a diseased tissue.

9. The nucleic acid molecule according to any one of claims 1-8, **characterized in that** the disease-associated gene is selected from ataxin-2, SLN, SOD1, ATXN3, C9ORF72, HTT, VEGF, VEGFR, ANG1, ANG2, SARS-CoV-2, HBV, HCV, HIV, HPV, HSV, COX-2, TGF-beta, GBAI, PCSK9, LDL, KRAS, NKRAS, HRAS, EGFR, ATL, MET, PIK3CA, SHP1, SOX2, PTEN, TP53, Yap1, HIF1a, CDK1, CDK4, CDK6, NRF2, beta-Catenin, STAT3, Aurora A, CDH17, CXCR4, IDO1, CSF-1, NF-kB, NOTCH, SOX9, PD-L1, PD1, CTLA4, LAG3, TIGIT, COX-2, TGF-beta, VEGF, VEFGR, BRAF, KRAS, and EGFR genes.

10. The nucleic acid molecule according to any one of claims 1-9, **characterized in that** the small RNA molecule has a sequence as set forth in any one of SEQ ID NOs: 6, 8, 10, 12, 14, 16, 18, 20, 22, 24, 26, 28, 30, 32, 34, 36, 38, 40, 42, 44, 46, 48 or 50.

11. The nucleic acid molecule according to any one of claims 1-10, **characterized in that**:
1) the small RNA molecule has a sequence as set forth in SEQ ID NO: 6, and an RNA sequence produced by transcription of the compensatory sequence is as set forth in SEQ ID NO: 7;
2) the small RNA molecule has a sequence as set forth in SEQ ID NO: 8, and an RNA sequence produced by transcription of the compensatory sequence is as set forth in SEQ ID NO: 9;
3) the small RNA molecule has a sequence as set forth in SEQ ID NO: 10, and an RNA sequence produced by transcription of the compensatory sequence is as set forth in SEQ ID NO: 11;
4) the small RNA molecule has a sequence as set forth in SEQ ID NO: 12, and an RNA sequence produced by transcription of the compensatory sequence is as set forth in SEQ ID NO: 13;
5) the small RNA molecule has a sequence as set forth in SEQ ID NO: 14, and an RNA sequence produced by transcription of the compensatory sequence is as set forth in SEQ ID NO: 15;
6) the small RNA molecule has a sequence as set forth in SEQ ID NO: 16, and an RNA sequence produced by transcription of the compensatory sequence is as set forth in SEQ ID NO: 17;
7) the small RNA molecule has a sequence as set forth in SEQ ID NO: 18, and an RNA sequence produced by transcription of the compensatory sequence is as set forth in SEQ ID NO: 19;
8) the small RNA molecule has a sequence as set forth in SEQ ID NO: 20, and an RNA sequence produced by transcription of the compensatory sequence is as set forth in SEQ ID NO: 21;
9) the small RNA molecule has a sequence as set forth in SEQ ID NO: 22, and an RNA sequence produced by transcription of the compensatory sequence is as set forth in SEQ ID NO: 23;
10) the small RNA molecule has a sequence as set forth in SEQ ID NO: 24, and an RNA sequence produced by transcription of the compensatory sequence is as set forth in SEQ ID NO: 25;
11) the small RNA molecule has a sequence as set forth in SEQ ID NO: 26, and an RNA sequence produced by transcription of the compensatory sequence is as set forth in SEQ ID NO: 27;
12) the small RNA molecule has a sequence as set forth in SEQ ID NO: 28, and an RNA sequence produced by transcription of the compensatory sequence is as set forth in SEQ ID NO: 29;
13) the small RNA molecule has a sequence as set forth in SEQ ID NO: 30, and an RNA sequence produced by transcription of the compensatory sequence is as set forth in SEQ ID NO: 31;
14) the small RNA molecule has a sequence as set forth in SEQ ID NO: 32, and an RNA sequence produced by transcription of the compensatory sequence is as set forth in SEQ ID NO: 33;
15) the small RNA molecule has a sequence as set forth in SEQ ID NO: 34, and an RNA sequence produced by transcription of the compensatory sequence is as set forth in SEQ ID NO: 35;
16) the small RNA molecule has a sequence as set forth in SEQ ID NO: 36, and an RNA sequence produced by transcription of the compensatory sequence is as set forth in SEQ ID NO: 37;
17) the small RNA molecule has a sequence as set forth in SEQ ID NO: 38, and an RNA sequence produced by transcription of the compensatory sequence is as set forth in SEQ ID NO: 39;
18) the small RNA molecule has a sequence as set forth in SEQ ID NO: 40, and an RNA sequence produced by transcription of the compensatory sequence is as set forth in SEQ ID NO: 41;
19) the small RNA molecule has a sequence as set forth in SEQ ID NO: 42, and an RNA sequence produced by transcription of the compensatory sequence is as set forth in SEQ ID NO: 43;
20) the small RNA molecule has a sequence as set forth in SEQ ID NO: 44, and an RNA sequence produced by transcription of the compensatory sequence is as set forth in SEQ ID NO: 45;
21) the small RNA molecule has a sequence as set forth in SEQ ID NO: 46, and an RNA sequence produced by transcription of the compensatory sequence is as set forth in SEQ ID NO: 47;
22) the small RNA molecule has a sequence as set forth in SEQ ID NO: 48, and an RNA sequence produced by transcription of the compensatory sequence is as set forth in SEQ ID NO: 49; or
23) the small RNA molecule has a sequence as set forth in SEQ ID NO: 50, and an RNA sequence produced by transcription of the compensatory sequence is as set forth in SEQ ID NO: 51.

12. An expression vector, **characterized by** comprising the nucleic acid molecule according to any one of claims 1-11.

13. The expression vector according to claim 12, **characterized by** further comprising a promoter sequence operably linked to the nucleic acid molecule.

14. The expression vector according to claim 12 or 13, **characterized in that** the vector is selected from a plasmid vector, an adeno-associated viral vector, an adenoviral vector, a retroviral vector, and a lentiviral vector.

15. A host cell, **characterized by** comprising the nucleic acid molecule according to any one of claims 1-11 or the expression vector according to claim 12, 13, or 14.

16. A method for producing a small RNA molecule, **characterized by** comprising culturing the host cell according to claim 15 and optionally isolating the small RNA molecule.

17. A method for producing an exosome comprising a small RNA molecule, **characterized by** comprising culturing the host cell according to claim 15 and isolating the exosome from a culture supernatant.

18. Use of the nucleic acid molecule according to any one of claims 1-11, the expression vector according to claim 12, 13, or 14, or the host cell according to claim 15 in the preparation of a small RNA molecule.

19. A pharmaceutical composition, **characterized by** comprising:
1) the expression vector according to claim 12, 13, or 14, or the host cell according to claim 15; and
2) a pharmaceutically acceptable carrier.

20. Use of the expression vector according to claim 12, 13, or 14, or the host cell according to claim 15 in the preparation of a medicament for treating a disease, **characterized in that** the disease is related to an abnormal expression or mutation of a gene.

21. A method for treating a disease in a subject, **characterized by** comprising administering to the subject a therapeutically effective amount of the expression vector according to claim 12, 13, or 14, or the host cell according to claim 15, wherein the disease is related to an abnormal expression or mutation of a gene.
